# EUROPEAN PATENT APPLICATION

(11) **EP 3 851 054 A1**
(43) Date of publication of application: **21.07.2021**
(21) Application number: 20152518.5
(22) Date of filing: 17.01.2020
(51) Int. Cl.: A61B 17/00

(54) **OCCLUDER DEVICE**

(71) Applicant: Universität Zürich, 8006 Zürich (CH); Teqable AG, 8810 Horgen (CH)
(72) Inventor: DAVE, Hitendu, 8044 Zürich (CH); SCHINDLER, Julia, 8810 Horgen (CH); MEDRICKY, Petr, 6045 Meggen (CH); FLÜCKIGER, Hans, 8618 Oetwil am See (CH); KINDLER, Gereon, 8002 Zürich (CH)
(74) Representative: Kleine, Hubertus

(57) **Abstract**

An occluder device (1, 11, 21, 31, 41, 61) for the closure of a hole in the tissue (5, 15, 25) of an organ, especially for the repair of a ventricle septal defect,
wherein the occluder device (1, 11, 21, 31, 41, 61) further comprises at least one supporting structure (2, 12, 22, 32, 42, 52, 62),
wherein the supporting structure (2, 12, 22, 32, 42, 52, 62) comprises a circumferentially outer element (6, 16, 26, 36, 46, 56, 66) as a stop against a first side of the organic tissue (5, 15, 25, 35, 45) surrounding the hole, wherein said circumferentially outer element (6, 16, 26, 36, 46, 56, 66) defines a plane and a center axis perpendicular to said plane and
wherein the occluder device (1, 11, 21, 31, 41, 61) comprises anchoring segments (4, 14, 24, 34, 44, 64) wherein a connection of the anchoring segments (4, 14, 24, 34, 44, 64) with the tissue (5, 15, 25, 35, 45) can be made either by sewing, stapling and/or by a rear grip with the tissue (5, 15, 25, 35, 45) at the opposite side of the tissue (5, 15, 25),
characterized in that
the supporting structure (2, 12, 22, 32, 42, 62) comprises an element which is positioned closer to the center axis than the circumferentially outer element (6, 16, 26, 36, 46, 66), wherein said element can be moved relative to the circumferentially outer element (6, 16, 26, 36, 46, 66) along the center axis by generating a restoration force for holding the circumferentially outer element (6, 16, 26, 36, 46, 66) in place during a contraction and/or expansion of the organ.

## Description

### Technical Field

The subject of the present invention is an occluder device according to the preamble of claim 1, which can be used to repair a Ventricle Septal Defect (VSD). This defect is located in the septum separating the right and the left ventricle of the heart. It is most often single, located in the perimembranous area, which is the upper part of the septum abutted by the tricuspid and aortic valves, hence called a perimembranous VSD. It can however also be a muscular VSD, an outlet VSD, or multiple swiss-cheese muscular VSD depending on their location, margins and the multiplicity. Additionally VSD may coexist as a part of more complex defects.

Many of these VSD need to be closed, optimally during the first 6 months of life. The longer they are left unattended, the higher is the risk of developing pulmonary hypertension. A stage is reached when left unattended, when the pulmonary hypertension gets fixed due to sclerosis of the pulmonary vascular bed leading to Eisenmenger Syndrome: a point when complete VSD closure is prohibited.

### Background Art

VSD closure has been successfully performed since 1954, when it was amongst the first intra-cardiac operations performed by Walton Lillehei at University of Minnesota Hospitals, Minneapolis, USA. Since then the VSD closure with a biological/prosthetic patch using a Heart Lung Maschine has become a routine operation with very good results. The operative technique has hardly changed since decades.

The present state of the art technique involves supporting the circulation using extra-corporeal support (Heart Lung Maschine) which needs to be filled with blood to prevent extreme haemodilution and to maintain the natural homeaostasis. The heart needs to be stopped by administering cardioplegia, for a variable duration ranging up to 60 minutes to allow for careful suturing of the patch to the VSD.

The suturing also subjects the heart to a risk of atrioventricular block (due to injury to the bundle of HIS). The bundle of HIS runs within the septum on the posteroinferior margin of the VSD. 1-3% of patients undergoing surgery for VSD closure develop permanent AV Block requiring insertion of a permanent pacemaker which subjects the child to a cycle of repeated reoperations, risk of infection, endocarditis, etc.

A method to close a VSD is using a dumbbell shaped device which is often called 'Amplatzer'-device delivered transvenously using a catheter.
Catheter is maneuvered into the heart using X ray guidance. This method is applicable in a minority of VSD closure 'especially mid muscular VSD' and in elderly patients above 10 kg. The majority (> 95%) of VSD closure are therefore performed surgically. The 'Amplatzer'-device is voluminous and has no elasticity. It is further not possible to close a VSD near the heart valve. A recent development is to use the 'Amplatzer device surgically to close the VSD. This technique is being stretched to use in children under 1 year of age; but continues to be propagated only by a few centers in China, albeit with good results. Said device in introduced through the vessels, which is however very complicated or even impossible with infants.

A further device for the closure of a VSD is an occluder provided by the Carag AG. This occluder is also dumbbell-shaped and has no elasticity or flexibility. Therefore it is similar to the 'Amplatzer'-device.

A further state of the art is the closure of a VSD by a surgical patch. It involves inserting a textile patch and putting it in place using numerous stitches (each on the septum as well as the patch). The operation has to be done by very experienced and trained surgeons. A patch is a simple way for the closure of a VSD. It is well accepted by the body and can be overgrown with tissue. However surgical patch has no elasticity and can be a hindrance for the movement of the heart. The time of the surgical operation and for the recovery is long. The numerous stitches near the nerve pathway have the danger of an AV-Block. Due to the low elasticity of the patch, the patch does not participate in the growth of the organ. The pressure in the left ventricle is almost 5 times greater than in the right ventricle during systole (during contraction). As a consequence, the patch can actually bulge away towards the right ventricle when the rest of the left ventricular muscle is contracting, thus to a small extent negating the contractile effect at the base of the heart. Depending on the size and site of the VSD, as well as the occurrence of right bundle branch block (often seen after conventional surgical closure), the consequences can become relevant.

Further a Hernia Patch with the trademark name "Ventralex" is reported in the literature. This Hernia Patch however is not suited for cardiovascular defects.

### Object of the invention

Starting from the state of the art mentioned above, it is an object of the current invention to reduce operation time for the attachment of a patch in order to repair a Ventricle Septal Defect. Further to this it is an object of the current invention to reduce the recovery time of a patient.

The aforementioned occluder devices in the state of the art are either voluminous and shape, have no elasticity and/or are time demanding during the operation. They can also be not suitable or have other disadvantages as the danger of the formation of an AV-block.

It can be a further object of the invention that the patch is applicable by a method, which does not differ substantially from the established methods for the application of patches in the state of the art.

It is a further object of the invention to generate a better tightness of the patch compared to patches mentioned in the state of the art.

A further object of the invention is that the application of the patch can be provided with high quality and less training for the surgeons.

### Disclosure of the invention

The current invention overcomes the aforementioned disadvantages with an occluder device with the features of claim 1.

An inventive occluder device can be used for the closure of a hole in the tissue of an organ, especially for the repair of a ventricle septal defect.

The occluder device may preferably comprise at least one covering material. Said covering material can partly or completely cover the surface of the defect. When there is a partly covering the covering material should at least cover the majority of the surface, preferably at least 65% of the surface of the defect. The covering can preferably be more than 90% or even more than 95%. Also a complete covering is desired. The free space of less than 35%, preferably less than 30%, can be provided by openings of preferably less than 3 mm diameters. These openings can be covered by natural tissue material.

The occluder device further comprises at least one supporting structure which can for example support a sewing material such as strings for the attachment of said occluder device. The supporting structure may be preferably adapted for supporting the covering material, however the occluder can also be used without said covering material and with said supporting structure only.

The supporting structure can preferably be made of metal and/or polymer material. It can more preferably be made of a metal sheet with less than 1 mm, preferably less than 0.5 mm, more preferably less than 0.3 mm thickness, most preferably between 0.05 and 0.25 mm. The material can be a metal, such as Nitinol, spring steel and/or stainless steel. Stainless spring steel or 1.4404-steel is more preferred. Especially thin edges are of advantage for the overgrowth of the occluder by organic tissue.

The supporting structure comprises circumferentially outer element as a stop against a first side of the organic, especially human, tissue surrounding the hole, wherein said outer circumferential element defines a plane and a central axis perpendicular to said plane. The circumferentially outer element can be an outer ring, wherein the ring is not restricted to a circular shape, but can also have oval, triangular, rectangular, hexagonal or any other suitable shape. The circumferentially outer element can also have only ring segments, which are connected to one another by connection segments. The outer circumferential element might also have or comprise a netlike structure, a three-dimensional adjusted structure, spokes-like structure, a form-adjusted structure, a spiral structure and/or a labyrinth structure.

The supporting structure provides a support to the covering material, such that the covering material is spread out and held in said plane. The supporting structure can be flexible, so that the occluder device can be bend or folded or otherwise introduced through the hole to be covered with. Upon bending or other deformation, the supporting structure develops a restoration force, so that it will tend to form back to an initial form, especially by reversible elastic deformation. Elasticity and/or flexibility may have further advantages for the tightness of the occluder.

The element which is positioned closer to the center axis than the circumferentially outer element can be moved relative to the circumferentially outer element along the center axis preferably between 0 - 6mm, more preferably between0.5 - 4mm, most preferably between 1 mm - 3mm.

The occluder device comprises anchoring segments can extend either from the covering material if such a covering material is provided or from the supporting structure such that a connection of the anchoring segments to the tissue can be made either by sewing, stapling and/or more preferably by a rear grip with the tissue at the opposite side of the organic tissue.

With the rear grip, the circumferential outer element abuts at the one side of the organic tissue and the anchoring segments are positioned at said opposite side of said organic tissue. For the positioning of said occluder, it is possible that the circumferential outer element will be introduced through the hole of said organic tissue, while the anchoring segments remain mostly outside of said hole.

In the case that the organ is the heart, the circumferential outer element, which supports the covering material, is positioned at the pressure side of the heart, which means inside the organ, while the anchoring segments are positioned on the other side of the dividing septum.

More preferred from the methods of connection is the sewing. The anchoring segments are wires, strings or straps that are connected or guided near the center of the outer circumferential element and are meant to extend along the central axis through the defect, respectively through the hole, bent away from the hole and are then connected at the opposite side of the hole to the tissue. The connection can be either a fixation either by sewing or, less preferred, by stapling. Biodegradable staples and/or sewing yarn are known in the art and can be used for this application.

If the connection is done by sewing, the inventive occluder reduces the amount of stitches to up to less than 30% compared to the patches of the state of the art. However sewing is not needed at all.

Alternatively or optionally to the sewing or stapling a fixation of the Occluder device can be achieved by rear grip of the tissue by the anchoring elements, such as wire arms that press with a distal end against the tissue.

Other suitable anchoring segments might be strings, yarn or threads, such as yarn threads, clamping legs, a clamping ring, one or more hooks, an umbrella-supporting structure, a spiral structure and/or knots.

In addition to the outer circumferential element, the inventive supporting structure comprises at least an element, which is positioned closer to the center axis than the outer circumferential element. Said element can be moved relatively to the outer circumferential element along the center axis by generating a restoration force for holding the outer circumferential element and the covering material in place during a contraction and/or expansion of the organ. This restoration force can be transferred by the anchoring segments to the outer circumferential element and/or the covering material, such that the outer circumferential element, the covering material or preferably both are pressed against the hole. This is a further difference to common patches and plasters, such as hernia patches.

Due to a preferred flexibility of the aforementioned element along the center axis, this element can be positioned inside the hole while the outer circumferential element is positioned next the hole at the surface of the tissue. Thereby the element can be a stop for a horizontal (lateral) movement or displacement of the occluder device to the center axis.

Further advantageous embodiments of the invention are subject-matter of the sub-claims.

The element of the supporting structure can be formed as a cantilever or spring arm and/or as an inner ring having a smaller diameter of the outer circumferential element. This inner ring can preferably have a center, which is also positioned at the center axis defined by the outer circumferential element. The cantilever arm can have a curved, preferably convex or concave structure, when it is mounted and might be flat in a not mounted situation. He can preferably decrease in width towards his distal end. Such a decrease in width means that the flexibility of the cantilever arm increases. At the distal end a cantilever arm can preferably be provided with a guiding bar, where an anchoring segment can be guided from a lateral extend towards the direction of the center axis and the defect. An anchoring segment could be laid up at the distal end of the cantilever arm, especially at the guiding bar. By applying a pulling force at the anchoring segment, the cantilever arm can be bend towards the hole. The defect, respectively the hole in the organ can move, increase or decrease in width and length upon expansion or contraction. Therefore the support structure with the covering material is only pressed against one side of the defect and fixed by anchoring elements, so that the occluder device is able to move to a certain amount when a force is applied. The outer circumferential element and the at least one element of the supporting structure additional to the outer circumferential element help to compensate and redirect pulling forces applied in lateral direction or parallel to the center axis.

A compensation of forces can be further achieved when the inner ring is preferably connected to the outer circumferential element by spring arms, preferably made of spring steel, nitinol or an elastomeric polymer and/or biodegradable material. Said spring arms can preferably also have a curved form, more preferably concave or convex form when mounted and planar form when not mounted.

The Occluder device can comprise a disc shaped segment for covering the defect. The anchoring segments can extend from the center of the disc shaped segment. The disc shaped segment can preferably be formed by the supporting structure having a disc shape and covered, especially pocketed, by the covering material.

The supporting structure is pocketed by a mantle or envelope formed by the covering material.

The cantilever arm or spring arms are preferably curved. More preferred they can have a concave or convex form.

The spring arm can be provided with interstitial space segments in the form of ring segments for the compression in longitudinal direction of the spring arm. The interstitial space segments can be arranged in line in radial direction towards the center axis defined by the outer circumferential element. The lined-up interstitial space segments can preferably decrease in size towards the center axis.

The covering material can preferably comprise a knitted, woven, jersey or crochet material or a lace-making structure.

The supporting structure can preferably made of silicone, nitinol-metal, 1.4404 stainless steel and/or spring steel and/or biodegradable material.

The anchoring segments can preferably made of sewable textile straps, more preferably made of the covering material. The Occluder device can comprise at least 2, preferably 3 or 4, sewable textile straps as anchoring segments. The provision of anchoring segments can reduce significantly the time for the fixation of the Occluder device compared to a circumferential sewing seam for the connection of a textile patch. It can be easily introduced and positioned at the place of the defect.

The cover material can comprise polyfluorinated material, preferably PTFE (Polytetrafluoroethylene), more preferred ePTFE, PET (Polyethylene terephthalate), PES (Polyethersulfone), Polypropylene, Fiber-Material and/or biological Cell-Material.

The occluder device is provided with a sealing lip, so that a sealing between the tissue and the occluder device, more preferably the disc-shaped segment, can be achieved.

The inner ring can be connected to the outer circumferential element by spring arms, preferably made of spring steel or an elastomeric polymer material.

The inner ring can be connected to the outer circumferential element by multiple strings. Said strings can be the covering material. They can preferably be elastic strings, more preferably stretchable strings, especially stretchable Polyamide-strings, such as Stretch-Nylon.

### Brief Description of the drawings

Some advantageous embodiments for inventive occluder devices are further explained in detail below together with drawings. Specific parts of the different embodiments can be understood as separate features that can also be realized in other embodiments of the invention. The combination of features described by the embodiment shall not be understood as a limitation for the invention:
- Fig. 1: a schematic view on parts of a first inventive embodiment of an occluder device;
- Fig. 2: a cross-sectional view of the occluder device of Fig. 1 in intended position in a hole of a heart;
- Fig. 3: a schematic view of a second embodiment of a supporting structure of the occluder device;
- Fig. 4: a schematic view of a second embodiment of a supporting structure of the occluder device with segments of further parts of the occluder;
- Fig. 5: a cross-sectional view of the occluder device mentioned in Fig. 3 and 4 in intended position in a hole of a heart;
- Fig. 6: a schematic view of a third inventive embodiment of parts of an occluder device;
- Fig. 7: a cross-sectional view of the occluder device of Fig. 5 in intended position in a hole of a heart;
- Fig. 8: a schematic view of a fourth inventive embodiment of parts of an occluder device;
- Fig. 9: a schematic view of a fifth inventive embodiment of an occluder device;
- Fig. 10: a schematic view of a sixth inventive embodiment of an occluder device;
- Fig. 11: a schematic view of the position of an occluder device at the heart;
- Fig. 12: a schematic view of a variation of the parts shown in Fig. 1;
- Fig. 13: two schematic view of a seventh inventive embodiment of an occluder device; and

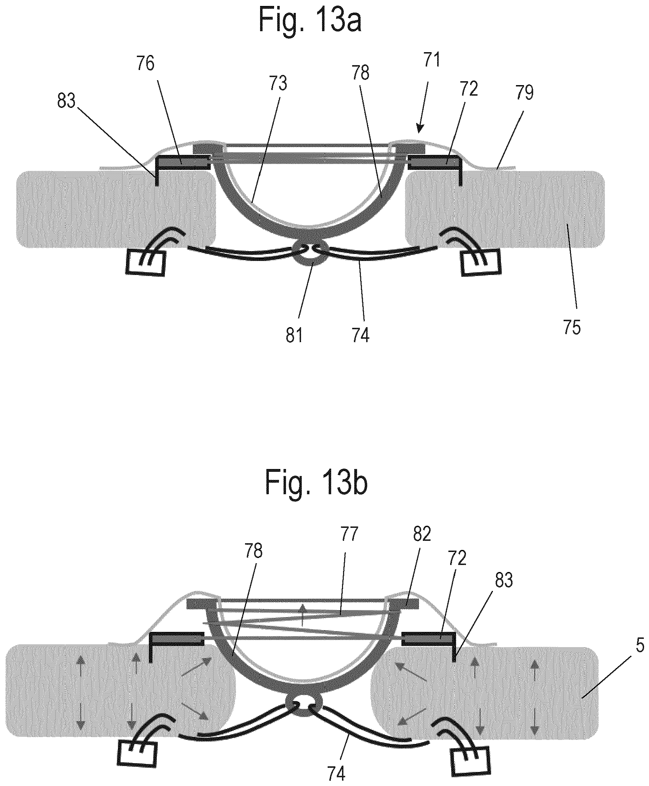

- Fig. 13: two schematic view of a eighth inventive embodiment of an occluder device.

### Mode for Carrying out the invention

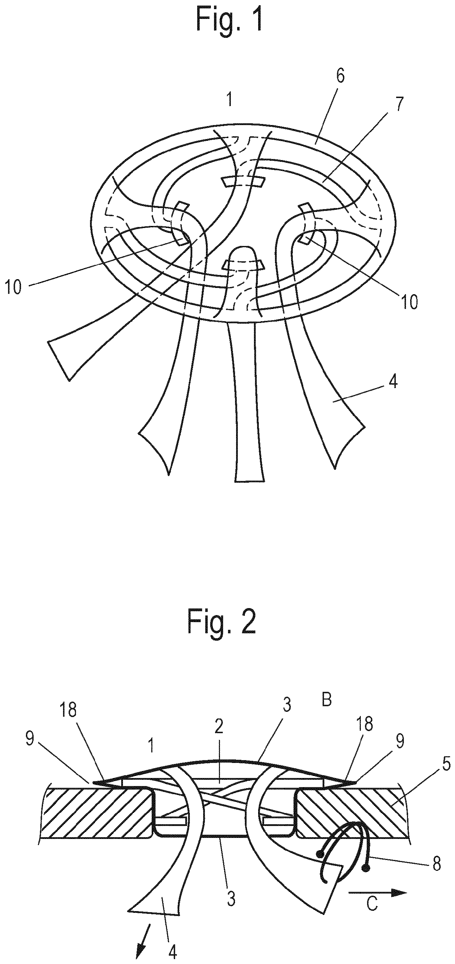

An occluder device 1 according to the invention is shown in Fig. 1 and 2. It comprises a supporting structure 2, a mantle or envelope 3 covering the supporting structure 2 and anchoring segments 4 for the connection to the tissue preferably either by sewing or a rear grip of the organic, especially human, tissue 5 at the opposite side of an organ which can be a heart. Additional to the use for the treatment of VSD the occluder device can be used for the repair of other organs with similar defects. A defect is in most cases a hole having a longitudinal axis at the center of the hole and defining an axial direction. Said longitudinal axis can be parallel to the insertion direction of the occluder device 1 in the defect, respectively the hole.

The supporting structure 2 has a disc-shaped form with disc shaped segments 18 and preferably consists of a flexible material which can develop restoring forces upon complete or partial deformation of said supporting structure 2. Such a material can preferably be spring steel. The disc-shaped supporting structure 2 will preferably be made of a material, resistant to millions of heart beats that it will be subjected to during a life-time. The supporting structure 2 of Fig. 1 comprises an outer circumferential element 6 defining a plane and cantilever arms 7 extending in a curved, preferably convex or concave, shape on said plane from the outer circumferential element 6 towards the center of the element 6, more specifically to the center axis A which passes through the center of the outer circumferential element 6 and which extends perpendicular to the plane defined by the ring. A guiding element 10, such as a guiding bar, for interaction with the anchoring segments 4 is provided at the distal end of each cantilever arm 7. The anchoring segments 4 can be redirected from a radial direction towards an axial direction by said guiding element 10, wherein the cantilever arms 7 will be bend out of plane if a pulling force is applied on the cantilever arms 7. In an installed position of the occluder device, the cantilever arms are always bend out of plane, in order to avoid movements of the occluder device perpendicular to the axis defined by the defect.

The connection between the outer circumferential element and the cantilever arms can be seamless. The supporting structure can preferably be produced from a sheet, such as a sheet of metal, by Laser-cutting, etching, stamping or stretching.

The disc-shaped supporting structure might have a preferred thickness of less than 0.5 mm. For this thickness there is an optimal flexibility of the cantilever arms 7 and for the whole supporting structure 2. The width of the cantilever arms can be reduced from the outer circumferential element 6 to the center of the circumferential element 6.

The mantle or envelope 3 completely or partially covers the supporting structure 2 so that a membrane for covering the defect area is established. It might be sufficient that the defect is reduced to a certain diameter such that the tissue can grow over the membrane and close the defect. Therefore the membrane formed by the mantle or envelope 3 might still have pores, minor recesses or the like of less than 3mm. The material of the mantle or envelope can preferably be a sealing material such as polyfluorinated material, especially PTFE (Polytetrafluoroethylene). In a preferred embodiment the PTFE-Material might be an expanded, especially stretched, so-called ePTFE, such as Gore- Tex-Material. Additionally or alternatively the material for the mantle or envelope 3 can be PET (Polyethylene terephthalate), PES (Polyethersulfone), Polypropylene, Fiber-Material and/or biological Cell-Material. The material should preferably cause minimum friction or damage to blood elements and will align the left ventricle to the aorta as much as is possible. The material can be provided with functional layers or fibers with growth-promoting compounds for cellular growth, such as magnesium.

The anchoring segments 4 in Fig. 1 can be attached either to the supporting structure 2 or to the cover material, which can be for example a monolayer, a mantle or envelope 3, or cantilever arms 7. The anchoring segments are preferably made of sewable material, for example a textile material, such as the material of the mantle or envelope 3 mentioned above. The anchoring segments 4 can be formed as straps, with sufficient length to extend from the supporting structure 2 or from mantle or envelope 3 through the defect such as a hole and laid against the outer side of the organ for sewing. Sewable material might also be a plastic material which is provided with puncture holes where a sewing needle can pass through, such that the anchoring segment 4 and therefore the occluder device 1 can be connected to the organ at this point or area of connection.

An additionally or alternatively to the aforementioned sewing connection the anchoring segments 4 might be a rear grip at the opposite outer side of the organ such as the heart. The anchoring segments in this case might be a bendable material such as a wire segment extending preferably from the support element 2.

The anchoring segment 4 can form together with the outer circumferential element 6 a u-shaped section such that the outer circumferential element 6 can bear against an inner surface of the organ and the anchoring segment 4 extend through the defect, such as a hole, and bears against the outer surface of the organ. To provide flexibility for the surgical closure element 2 to pass through the defect of the organ, the anchoring segments 4 can be formed in this case in the form of prongs.

The closure device shown in Fig. 1 and 2 envisage a 'step-up'-approach to close all types of VSD (congenital as well as acquired, isolated as well as those occurring as part of complex lesion, single as well as multiple), using the occluder device, especially in a semi-automated intelligent manner.

The use of the single ultra low-profile disc-shaped closure device, is able to close the VSD from the left ventricular (LV) side. In the case of an application in the heart the occluder can provide an enhanced tightness while blood pressure is applied on said occluder.

By sewing 1 - 4 straps to the tissue, these straps can stitched to the safe locations on the right ventricular (RV) side of the septum in a stretched position recreating the diastole. The safe location of the 1-4 straps is meant to reduce the number of stitches by 70-95% and to ensure minimizing and/or prevention of heart block and thus the need for permanent pacemaker. Fig. 1 thus shows a connection of the straps 4 and the outer side C of the tissue 5 of a heart by sewing yarn 8.

The tension on the straps connected to the tissue is meant to translate the concentric motion of the VSD into an inward 'give-way' motion of the cantilever arms 7. This is thought to give little contractile effect to the device. In any case, it would prevent a paradoxical movement present with a flaccid patch.

The procedure for placing and fixing the occluder device 1 can have a significant shortening of time compared to normal operation time. This would naturally lead to a shortened cardiopulmonary bypass time. The use of the occluder device can make the surgery safe and acceptable to the surgical, referral, para-medical and/or patient/family community. Also the time of recovery is significantly reduced.

The occluder device is deployable trans-ventricular on a beating heart. The ultimate goal of curing patients born with a VSD would lay in regenerating a contractile muscular septum in place of the defect. While it is known that many of the structures taking shape and function during embryogenesis benefit from local signaling of flows.

When the occluder device is used for neonates and young infants, which are supposed to possess totipotent cells capable of cardiac regeneration in the early weeks and months of life, the occluder device can be grown together or overgrown with said cells.

The occluder device can provide a complete closure of shunts in majority of patients, as well as no significant distortion of aortic and tricuspid valve morphology and function. It can especially used for realizing a beating heart VSD closure.

During a cardiac cycle the blood-pressure varies with the contraction, ejection relaxation and filling of the heart. Since different parts of the heart are contracting and relaxing at different time of a cardiac cycle, contractions may be longitudinal and/or concentric in the region of the defect. Therefore the current invention provides a solution with the disc-shaped supporting structure 2 with cantilever arms 7 having different designs to transform the kinetic energy which is applied during the cardiac cycle to the occluder device into movements that do not effect the sealing of the occluder device or the fixation of said device.

The single disc-shaped supporting structure 2 covered by the mantle or envelope 3 forms a disc-shaped segment 18 of the Occluder device. The support structure 2 and the disc-shaped segment 18 are pressed against or abut against the inner side of the tissue 5 when the blood pressure is high. At the edge of the supporting structure 2, the mantle or envelope 3 or the outer circumferential element (6) may have a sealing lip 9. The sealing lip can be provided by two connected layers of the mantle or envelope 3, that are welded together thus forming a thicker layer compared to the layer which covers one side of the supporting structure 2. In the case of a cover instead of a mantle or envelope, where the cover covers the structure from one side only, the sealing lip 9 can also be formed from one single layer. This sealing lip is pressed against the inner side B of the tissue 5 of the organ, such as the heart, either by the blood pressure or by the restoration force generated by the supporting structure 2.

When the blood pressure is low, the disc-shaped supporting structure 2 is held in place by the anchoring segments 4 connected to the opposite side of the tissue.

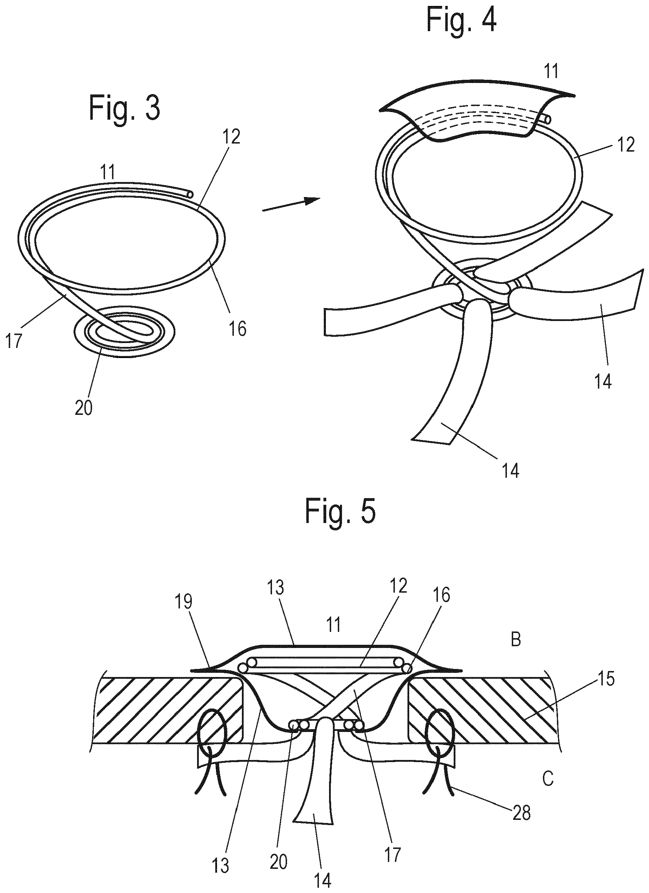

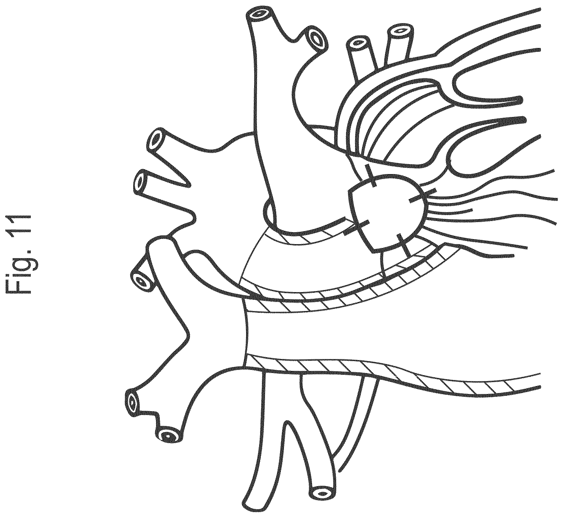

Fig. 3-12 disclose further embodiments for an inventive occluder device, having to a certain extend the same advantages as the embodiment described in Fig. 1 and 2. Preferred Materials for the different elements of the occluder devices, unless described otherwise, can be considered to be the same as in the embodiment of Fig. 1 and 2.

Fig. 3, 4 and 5 show a second embodiment for a occluder device 11 having a supporting structure 12, a mantle or envelope 13 covering the supporting structure 12 and anchoring segments 14 for the connection to the tissue preferably either by sewing or a rear grip of the tissue at the opposite side to the position of the supporting structure 12.

The supporting structure 12 can have a disc-shaped or conical form and comprises an outer circumferential element 16 defining a plane. Although it can be delivered as a disc, in the final position at the defect the support element of Fig. 3 and 4 has a conical form. It further comprises a smaller inner ring 20 positioned concentrically to the outer circumferential element 16 but preferably out of plane compared to the plane of the outer circumferential element, Both rings 16 and 20 are connected by at least one or more spring arms, such as spring wires or metal arms made of spring steel or spring arms made of suitable plastic material. These arms 17 have preferably a convex or concave form and extend this way from the outer circumferential element 16 to the center axis which passes through the center of the outer circumferential element and which extends perpendicular to the plane defined by the ring.

The support element 12 is pocketed in a mantle or envelope 13. The mantle or envelope can be provided with a sealing lip 19, similar to Fig. 1 or 2. Anchoring segments 14 in the form of sewable straps extend from the inner ring 20 to the outer side C of the tissue and are connected with the tissue 15 at these areas by yarn 28 or similar material. The outer circumferential element, just as in Fig. 1 and 2, works as a mechanical stop that limits the axial movement from one side of the occluder device 11 inside the defect, especially inside the hole. The axial movement from the other side is hindered due to the anchoring segments 14. The conical shape extending in axial direction in the defect can hinder the movement of the occluder device 11 in perpendicular direction to the longitudinal axis of the defect.

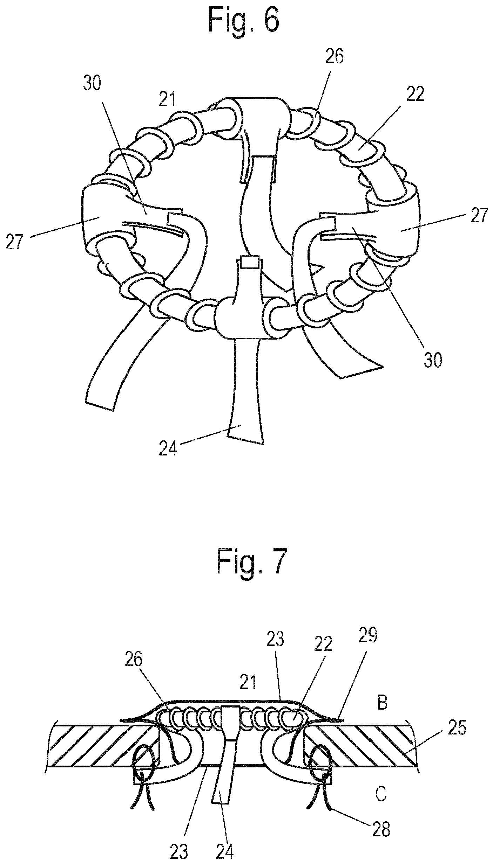

Fig. 6 and 7 show a third embodiment for an occluder device 21 having a supporting structure 22, a mantle or envelope 23 covering the supporting structure 22 and anchoring segments 24 for the connection to the tissue 25 preferably either by sewing or a rear grip of the tissue at the opposite side C to the position of the supporting structure 22 at the inner side B of the organ.

The supporting structure 22 comprises an outer circumferential element 26 defining a plane. The outer circumferential element 26 of Fig. 6 and 7 is a spiral spring ring or may comprise a spiral spring ring. The supporting structure 22 further comprises at least one, preferably at least two, more preferably at least four tilting arms 27, which have to be considered according to the invention as special spring arms, extending radially from the outer circumferential element 26 towards the center axis which passes through the center of the outer circumferential element 26 and which extends perpendicular to the plane defined by the outer circumferential element 26. The tilting arms 27 are fixed to the outer circumferential element 26. The fixation is provided in the current embodiment by sleeves 30 at one distal end of each tilting arm 27. At the second distal end the arms the anchoring segments 24 which are in the form of sewable straps are connected to the supporting structure.

The support element 22 is pocketed in a mantle or envelope 23. The mantle or envelope can be provided with a sealing lip 29, similar to the previous embodiments. The outer circumferential element 26 works as a mechanical stop that limits the axial movement of the occluder device 21 from one side of the tissue 25 inside the defect, especially inside the hole. Due to the form as spiral spring ring, the tilting arms can be tilted towards the defect, wherein the spring ring will develop restoring forces against the tilting operation. The axial movement from the other side is hindered due to the anchoring segments 24. The tilting arms 27 extending in the defect can hinder the movement of the occluder device 21 in perpendicular direction to the longitudinal axis of the defect.

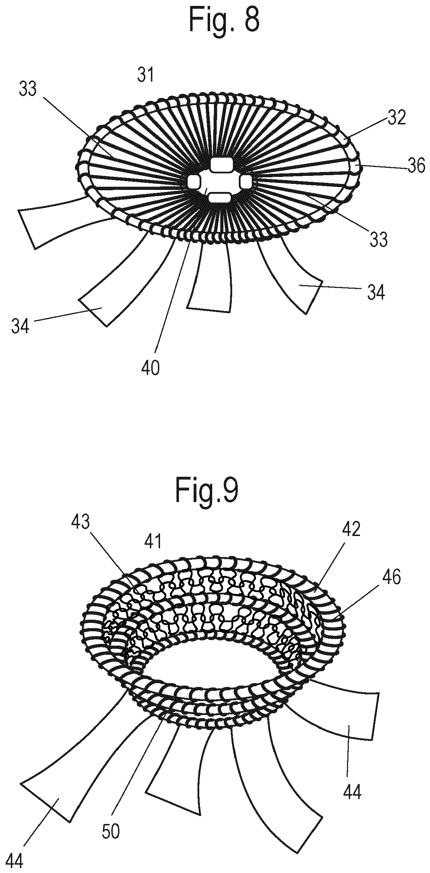

Fig. 8 show a third embodiment for an occluder device 31 having a supporting structure 32 and anchoring segments 34 for the connection to the tissue preferably either by sewing or a rear grip of the tissue at the opposite side to the position of the supporting structure 32.

The supporting structure 32 comprises an outer circumferential element 36 defining a plane, wherein a center axis perpendicular to said plane is defined, which passes through the center of the outer circumferential element 36. The supporting structure 32 further comprises an inner ring 40 with a center laying at the said center axis. The material of the outer and the inner ring 36, 40 can be made of metal wire, rubber or plastics.

The outer and the inner ring 36, 40 are connected by strings 33, such as yarn string, extending from the outer circumferential element 36 to the inner ring 40. The connection can be a knitted structure or a wrapped string structure. A mantle or envelope can be provided but is not necessary to cover the supporting structure 32 in this case, if the string distance is sufficiently small. In this case the arrangement of strings 33 forms the covering material and provides a restoration force if the inner ring 40 is moved along the center axis. Preferably the strings 33 are made of a stretchable material to enhance the flexibility of the material. Anchoring segments 34 in the form of sewable straps extend from the inner ring 40 to the outer side of the tissue and are connected with the tissue at these areas by yarn or similar material.

Fig. 9 show a fourth embodiment for a occluder device 41 having a supporting structure 42 and anchoring segments 44 for the connection to the tissue preferably either by sewing or a rear grip of the tissue 45 at the opposite side to the position of the supporting structure 42.

The supporting structure 42 in this case is an elastic polymer structure, having at least an outer and an inner ring 46, 50 and elastic textile material, preferably a knitted material, for connecting the rings. The supporting structure 42 in this case can be made of silicone or other flexible polymer material. The rings 46, 50 of the structure and the space between the said rings can be covered a crochet or lace-making or a knitted structure 43. In this example, a mantle or envelope is not needed. Similar to the embodiment of Fig. 7, the inner ring 50 of the supporting structure can be moved along the axis while the outer circumferential element can be fixed to the tissue 45.

The outer circumferential element can span the covering material and thus forming a disc-shaped segment for covering the defect in all the aforementioned embodiments.

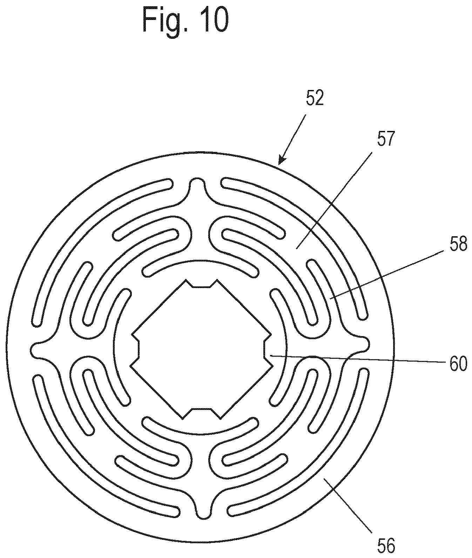

Fig. 10 show a sixth embodiment of a supporting structure 52 which can be exchanged with the supporting structure 2 of Fig. 1. The supporting structure 52 can have an outer circumferential element 56 provided with spring arms 57 extending from the outer circumferential element 56 towards a center axis to an inner ring 60 of the supporting structure 52.

The spring arm 57 is provided with interstitial space segments 58, such as slits, in the form of ring segments for the compression in longitudinal direction of the spring arm 56. The interstitial space segments 58 can be arranged in line in radial direction towards the center axis defined by the outer circumferential element 56. The lined-up interstitial space segments 58 decreases in size towards the center axis, whereas the slit width preferably remains constant.

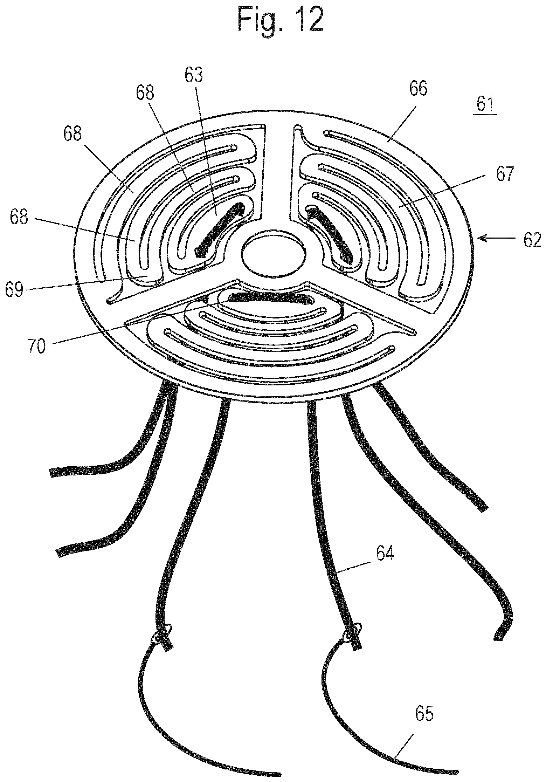

Fig. 12 discloses a further variation of the supporting structure 62 as a part of an occluder device 61, wherein the supporting structure 62 can be covered by a mantle or envelope, in the same way as the supporting structure 2 in Fig. 2.

In Fig. 12 the anchoring segments 64 are formed as strings, being connected to the guiding element 63 at the end of spring arms 67. In the embodiment of Fig. 12, the supporting structure 66 is provided with three of said planar spring arms 67. The supporting structure 66 has a plane shape and the guiding element 63 are each provided with openings 70 where the string is inserted such that both open ends of the string are oriented in the same side of the supporting structure 66. At the end of the strings needles 65 or connection strings are shown, which can be separate parts or parts of said inventive occluder. Each spring arm 67 comprises at least one, preferably 2-8 arc-shaped spring segments 68 and connected by u-shaped segments 69. The arc-shaped spring segments 68 have preferably the same curvature as the outer circumferential element 66.

Fig. 13 discloses a further variation of an occluder device 71 .The supporting structure 72 can comprise in this case multiple parts. An circumferentially outer element 76 which can be used as a stop against a first side of the organic tissue surrounding the hole, wherein said circumferentially outer element defines a plane and a center axis perpendicular to said plane. Said outer element 76 can be provided with optional separate anchoring elements 83 such as hooks that can extend into the tissue. The supporting element further comprises a hemisphere-like element 78, which is provided with one or more stop faces or a collar 82 which can interact with the surface of said outer element 76.

Anchoring segments 74 extend from the hemisphere-like element 78. These segments 74 may have the form of threads as shown in Fig. 13a and 13b.The structure 78 is provided with a eyelet 81 which defines the position of the anchoring segments 74 at said element 78.

The element 78 is connected to said supporting element 72 by means of one or more spring arms 77. During expansion of the hole in the tissue the hemisphere-like element can be pulled. During contraction the spring arms 77 generate a restoration force. During contraction of the organ as shown in Fig. 13b the occluder device is held in place by an interaction of the restoration force, the form of the element 78 and the anchoring elements 83.

The supporting structure 72, comprising the outer element 76, the hemisphere-like element 78 and the spring arm 77, is covered by a covering material 73, such as goretex. Said covering material can be provided with a sealing lip 79.

The hemisphere-like element 78 can be designed as a shell shape or as a full hemisphere. The anchoring segments 74 can be attached to the hemisphere-like element 78 or the spring elements 77.

Said collar 82 can be positioned around the equator of the element 78 as a stop so that the hemisphere-like structure cannot be pulled into the VSD opening by attaching the threads.

The supporting structure 72 can also contain hooks 83 to additionally position the occluder in the hole of the tissue. The movements are possible in the same directions as in Fig 1, but are primarily driven by the contraction of the muscles.

The occluder device 71 is placed on one side of the hole and also occupies the hole, so that the contractile force of the muscles comprising the margins of the hole generate a mild displacement of the central device element while the pressure on the other side maintains closure of the hole.

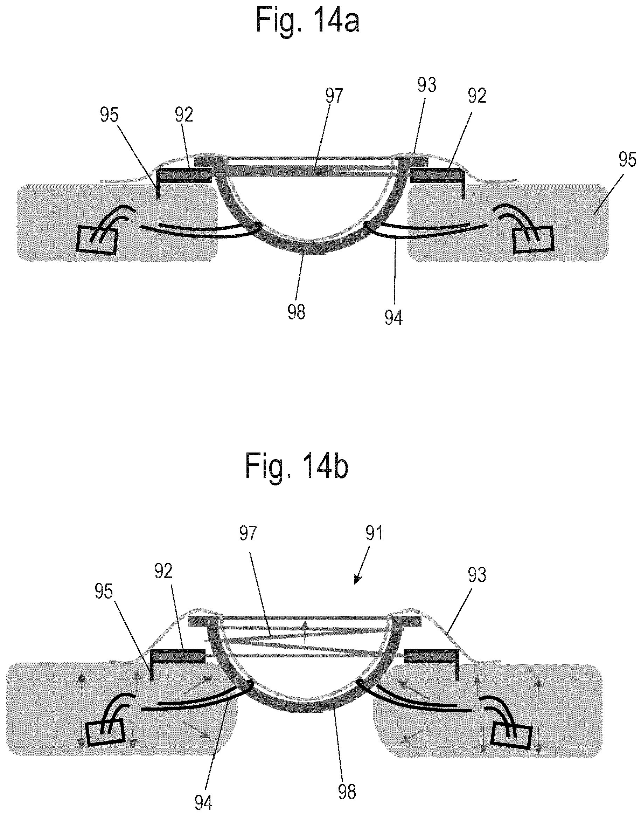

Fig. 14 discloses a further occluder device 91 with a support structure 92 and a covering material 93 covering said support structure 92. The support structure comprises a hemisphere like element 98 which can be displaced axially inside the hole of the tissue 95, as well as an outer circumferential element 96 and a spring element 97 for the connection of the elements 96 and 98. The anchoring elements 94 between the tissue 95 and the hemisphere-like element 98 can be provided as threads. As an option the outer element 96 may be provided with hooks for an additional attachment of the supporting structure 92.

The main difference between Fig 13 and 14 consists in the attachment of the anchoring elements 94 directly to the hemisphere like element 98 without an additional eyelet.

As explained before, the restoration force can be generated during a contraction or an expansion depending of the model of the occluder as shown in Fig. 1-14.

### List of references

- 1: occluder device
- 2: supporting structure
- 3: mantle,envelope or cover
- 4: anchoring segments
- 5: tissue
- 6: outer circumferential element
- 7: cantilever arms
- 8: yarn
- 9: sealing lip
- 10: guiding element
- 11: occluder device
- 12: supporting structure
- 13: mantle or envelope or cover
- 14: anchoring segments
- 15: tissue
- 16: outer circumferential element
- 17: spring arms
- 18: disc shaped segment
- 19: sealing lip
- 20: inner ring
- 21: occluder device
- 22: supporting structure
- 23: mantle, envelope or cover
- 24: anchoring segments
- 25: tissue
- 26: outer circumferential element
- 27: tilting arms
- 28: yarn
- 29: sealing lip
- 30: sleeves
- 31: occluder device
- 32: supporting structure
- 33: strings
- 34: anchoring segments
- 35: -
- 36: outer circumferential element
- 37: -
- 38: -
- 39: -
- 40: inner ring
- 41: occluder device
- 42: supporting structure
- 43: crochet or lace-making or knitted structure
- 44: anchoring segments
- 45:
- 46: outer circumferential element
- 47: -
- 48: -
- 49: -
- 50: inner ring
- 51: -
- 52: supporting structure
- 53: -
- 54: -
- 55: -
- 56: outer circumferential element
- 57: spring arms
- 58: interstitial space
- 59: -
- 60: inner ring
- 61: occluder
- 62: supporting structure
- 63: guiding element
- 64: anchoring element
- 65: needles
- 66: outer circumferential element
- 67: spring arm
- 68: arc-shaped spring segments
- 69: u-shaped spring segments
- 70: openings
- 71: occluder device
- 72: supporting structure
- 73: covering material
- 74: anchoring segments
- 75: tissue
- 76: outer circumferential element
- 77: spring arms
- 78: hemisphere-like element
- 79: sealing lip
- 80: -
- 81: eyelet
- 82: collar
- 83: additional anchoring elements

- 91: occluder device
- 92: supporting structure
- 93: covering material
- 94: anchoring segments
- 95: tissue
- 96: outer circumferential element
- 97: spring arm
- 98: hemisphere-like element
- 99: additional anchoring segments

- B: Inner side of the organ
- C: outer side of the organ

## Claims

1. An occluder device (1, 11, 21, 31, 41, 61) for the closure of a hole in the tissue (5, 15, 25) of an organ, especially for the repair of a ventricle septal defect,
wherein the occluder device (1, 11, 21, 31, 41, 61) further comprises at least one supporting structure (2, 12, 22, 32, 42, 52, 62),
wherein the supporting structure (2, 12, 22, 32, 42, 52, 62) comprises a circumferentially outer element (6, 16, 26, 36, 46, 56, 66) as a stop against a first side of the organic tissue (5, 15, 25, 35, 45) surrounding the hole, wherein said circumferentially outer element (6, 16, 26, 36, 46, 56, 66) defines a plane and a center axis perpendicular to said plane and
wherein the occluder device (1, 11, 21, 31, 41, 61) comprises anchoring segments (4, 14, 24, 34, 44, 64) wherein a connection of the anchoring segments (4, 14, 24, 34, 44, 64) with the tissue (5, 15, 25, 35, 45) can be made either by sewing, stapling and/or by a rear grip with the tissue (5, 15, 25, 35, 45) at the opposite side of the tissue (5, 15, 25),
**characterized in that**
the supporting structure (2, 12, 22, 32, 42, 62) comprises an element which is positioned closer to the center axis than the circumferentially outer element (6, 16, 26, 36, 46, 66), wherein said element can be moved relative to the circumferentially outer element (6, 16, 26, 36, 46, 66) along the center axis by generating a restoration force for holding the circumferentially outer element (6, 16, 26, 36, 46, 66) in place during a contraction and/or expansion of the organ.

2. An occluder device according to claim 1, **characterized in that** the occluder device (1, 11, 21, 31, 41, 61) comprises said at least one covering material for covering the surface of the defect, wherein the at least one supporting structure (2, 12, 22, 32, 42, 52, 62) is adapted to for supporting the covering material, and wherein the anchoring segments (4, 14, 24, 34, 44, 64) extending from a covering material or from the supporting structure (2, 12, 22, 32, 42, 52, 62) and wherein said element can be moved relative to the circumferentially outer element (6, 16, 26, 36, 46, 66) along the center axis by generating said restoration force for holding the circumferentially outer element (6, 16, 26, 36, 46, 66) and the covering material in place during a contraction and/or expansion of the organ.

3. An occluder device according to claim 1 or 2, **characterized in that** the element of the supporting structure (2, 12, 22, 32, 42, 52, 62) is a cantilever arm (7), a spring arm (17, 27, 57, 67) an inner ring (20, 40, 50, 60) or a combination of said arm and ring.

4. An occluder device according to claim 3, **characterized in that** the inner ring (20, 60) is connected to the circumferentially outer element (16, 56, 66), which can preferably have the form of an outer circumferential element, by spring arms (17, 57, 67), preferably made of spring steel, nitinol or an elastomeric polymer or biodegradable material.

5. An occluder device according to one of the preceding claims, **characterized in that** the occluder device (1, 11, 21, 31, 41, 61) comprises a disc shaped segment (18) for covering the defect or a hemisphere-shaped element (78, 98) to be partly introduced in the defect.

6. An occluder device according to one of the preceding claims, **characterized in that** the supporting structure (2, 12, 22, 32, 42, 62) is pocketed by a mantle or envelope (3, 13, 23) as a covering material or covered by a cover as covering material, especially from one side only, wherein material of the mantle, cover or envelope (3, 13, 23) preferably comprises polyfluorinated material, preferably PTFE (Polytetrafluoroethylene), more preferred ePTFE, PET (Polyethylene terephthalate), PES (Polyethersulfone), Polypropylene, Fiber-Material and/or biological Cell-Material.

7. An occluder device according to one of the preceding claims, **characterized in that** the cantilever arm (7) or spring arms (17, 27, 57, 67) has a curved, preferably a concave or convex form.

8. An occluder device according to one of the preceding claims, **characterized in that** the spring arm (57, 67) is provided with interstitial space segments (58) in the form of ring segments for the compression in longitudinal direction of the spring arm (57, 67).

9. An occluder device according to one of the preceding claims, **characterized in that** the covering material is a knitted or woven or felt or crochet material or a lace-making structure (43).

10. An occluder device according to one of the preceding claims, **characterized in that** the supporting structure (2, 12, 22, 32, 42, 62) is made of silicone or metal, preferably nitinol-metal, 1.4404 stainless steel and/or spring steel or biodegradable material.

11. An occluder device according to one of the preceding claims, **characterized in that** the anchoring segments (4, 14, 24, 34, 44) are sewable textile straps or strings, preferably at least 2, most preferred 3 or 4 sewable textile straps or strings.

12. An occluder device according to one of the preceding claims, **characterized in that** the occluder device (1, 11, 21, 31, 41) is provided with a sealing lip (9, 19, 29).

13. An occluder device according to one of the preceding claims, **characterized in that** the inner ring (40) is connected to the circumferentially outer element (36) by multiple strings (33), wherein the strings (33) are preferably the covering material.

14. An occluder device according to one of the preceding claims, **characterized in that** the element which is positioned closer to the center axis than the circumferentially outer element (6, 16, 26, 36, 46, 66) can be moved relative to the circumferentially outer element (6, 16, 26, 36, 46, 66) along the center axis preferably between 0 - 6mm, more preferably between0.5 - 4mm, most preferably between 1mm - 3mm.

15. An occluder device according to one of the preceding claims, **characterized in that** the circumferentially outer element (6, 16, 36, 46, 66) has a thickness of less than 0.5 mm, preferably less than 0.3 mm, most preferably between 0.05 and 0.25 mm.
